# EUROPÄISCHE PATENTSCHRIFT

(11) **EP 2 434 993 B1**
(45) Veröffentlichungstag und Bekanntmachung des Hinweises auf die Patenterteilung: **27.05.2015**
(21) Anmeldenummer: 10721718.4
(22) Anmeldetag: 28.05.2010
(51) Int. Cl.: A61F 2/82, B81C 99/00, A61L 31/02, C23C 14/00

(54) **VERFAHREN ZUR HERSTELLUNG EINES MEDIZINISCHEN FUNKTIONSELEMENTS MIT EINER FREITRAGENDEN GITTERSTRUKTUR**
METHOD FOR PRODUCING A MEDICAL FUNCTIONAL ELEMENT COMPRISING A SELF-SUPPORTING LATTICE STRUCTURE
PROCÉDÉ DE FABRICATION D'UN ÉLÉMENT FONCTIONNEL MÉDICAL POURVU D'UNE STRUCTURE DE GRILLE EN PORTE-À-FAUX

(30) Priorität: 29.05.2009 DE 102009023371
(43) Veröffentlichungstag der Anmeldung: 04.04.2012
(73) Patentinhaber: Acandis GmbH & Co. KG, 76327 Pfinztal (DE)
(72) Erfinder: QUANDT, Eckhard, 24226 Heikendorf (DE); ZAMPONI, Christiane, 24114 Kiel (DE); LIMA DE MIRANDA, Rodrigo, 24103 Kiel (DE)
(74) Vertreter: Kilchert, Jochen
(86) Internationale Anmeldenummer: PCT/EP2010/003277
(87) Internationale Veröffentlichungsnummer: WO 2010/136215

(56) Entgegenhaltungen:
- WO-A1-00/04204
- WO-A2-01/87371
- DE-A1-102006 029 831
- CHUN, Y; LEVI, DS; MOHACHANDRA, KP; TULLOCH, AW; RIGBERG, DA; VINUELA, F; VINUELA JR., F; CARMAN, GP: "Micro patterning processes for thin film nitinol endografts and evaluation of endothelialization in swine model" PROC. SPIE, Bd. 7650, Nr. 76502T, 8. April 2010 (2010-04-08), XP002599828

## Beschreibung

Die Erfindung betrifft ein Verfahren zur Herstellung eines medizinischen Funktionselements mit einer freitragenden Gitterstruktur gemäß dem Oberbegriff des Patentanspruchs 1. Ein derartiges Verfahren ist beispielsweise aus der WO 2008 000 467 A1 bekannt.

Für die Behandlung von Gefäßläsionen, insbesondere in zerebralen Gefäßen, mit Stents bzw. allgemein rohrförmigen Strukturen, ist es zweckmäßig, wenn diese eine relativ hohe Flexibilität aufweisen. Eine hohe Flexibilität begünstigt das Verhalten des Stents in eng gekrümmten Gefäßen. Dabei erfährt der Stent bei einer Krümmung eine Verlängerung, wobei sich die Bereiche des Stents, die sich auf der Außenseite der Gefäßkrümmung befinden, weiter dehnen bzw. strecken als die näher am Krümmungsmittelpunkt angeordneten Bereiche. Es besteht also ein Zusammenhang zwischen der Flexibilität im Sinne der Krümmungs- bzw. Biegefähigkeit und der Längenänderung bzw. maximalen Verlängerung des Stents.

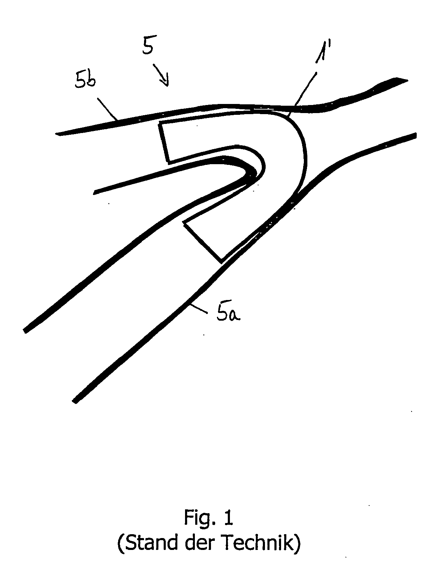

Fig. 1 verdeutlicht in schematischer Weise die der Erfindung zu Grunde liegende Problematik. Dabei zeigt Fig. 1 ein Blutgefäß 5, das einen ersten Gefäßabschnitt 5a und einen zweiten Gefäßabschnitt 5b aufweist, wobei zwischen dem ersten Gefäßabschnitt 5a und dem zweiten Gefäßabschnitt 5b eine Gefäßkrümmung mit einem relativ kleinen Winkel ausgebildet ist. Das bedeutet, dass die Gefäßkrümmung bzw. Gefäßwandkrümmung zwischen dem zweiten Gefäßabschnitt 5b und dem ersten Gefäßabschnitt 5a relativ eng ausgebildet ist. Um einen Stent in diesem Bereich zu platzieren, ist es daher erforderlich, dass der Stent dieser Gefäßkrümmung folgen kann. Die dafür erforderliche Krümmungsfähigkeit bzw. Flexionsfähigkeit des Stents wird im Rahmen der Erfindung als Flexibilität bezeichnet.

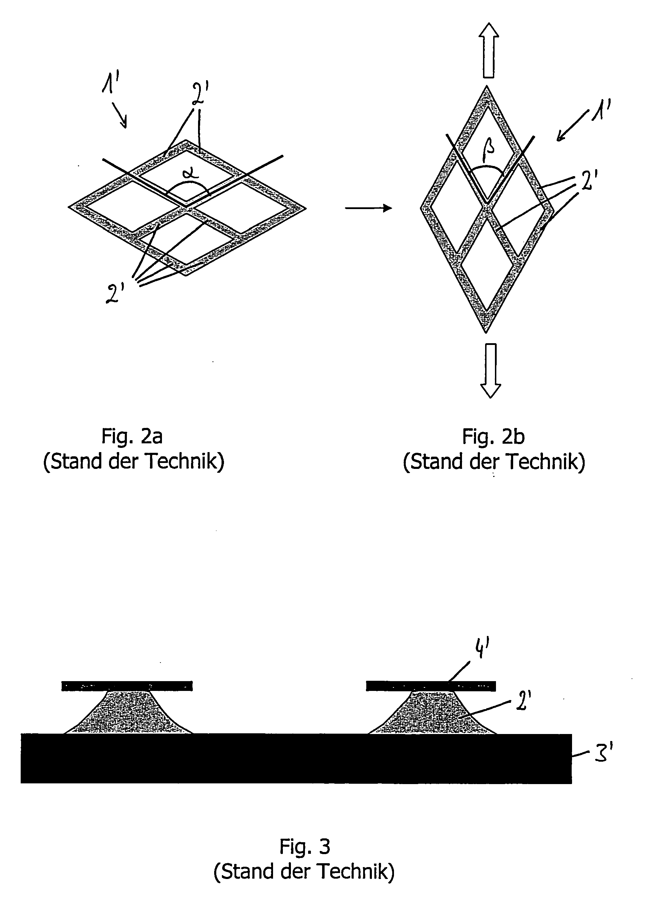

Bekannte Stents umfassen eine Gitterstruktur, die aus einem rohrförmigen Vollmaterial durch ein materialabtragendes Verfahren hergestellt ist. Die Gitterstruktur 1' umfasst, wie in den Figuren 2a und 2b dargestellt, eine Vielzahl von Zellen, die durch Stege 2' begrenzt sind. Dabei sind die Stege 2' unter einem Winkel miteinander verbunden und bilden eine rautenförmige Struktur. Durch Änderung des Rautenwinkels α, β wird bei bekannten Stents der Grad der Längenänderung bzw. die maximale Verlängerung bestimmt. Dabei ist es vorteilhaft, wenn der Rautenwinkel α im Ruhezustand relativ groß (Fig. 2a) und der Rautenwinkel β im gestreckten Zustand relativ klein ist (Fig. 2b).

Durch eine große Winkeldifferenz zwischen dem Rautenwinkel α im Ruhezustand und dem Rautenwinkel β im gestreckten Zustand besteht allerdings die Gefahr, dass sich das Stentmaterial an den Verbindungsstellen der Stege 2' plastisch verformt. Ferner bewirkt ein hoher Rautenwinkel α im Ruhezustand des Stents einen erhöhten Kraftaufwand, um den Stent in den komprimierten Zustand zu überführen. Dieser Problematik kann dadurch begegnet werden, dass das Materialvolumen an den Verbindungsstellen verringert wird, beispielsweise durch Reduktion der Stegbreite.

Bei Herstellungsverfahren für Stents, die auf einem Laserschneidprozess beruhen, bei dem die Gitterstruktur durch Materialabtragung aus einem rohrförmigen Vollmaterial geformt wird, sind die kleinstmöglichen Stegdimensionen aufgrund der thermischen Effekte entlang der Schneidkanten begrenzt.

Um kleinere Stegdimensionen zu erreichen, ist es bekannt, die Gitterstruktur des Stents durch ein Ätzverfahren zu formen, wobei vorzugsweise nass-chemische Ätzprozesse eingesetzt werden, die eine hohe Herstellungsgeschwindigkeit erlauben. Dabei wird, wie in Fig. 3 dargestellt, auf eine Substratschicht 3' eine Stegschicht 2" aufgebracht, die das Material der herzustellenden Gitterstruktur umfasst. Die Stegschicht 2" wird dabei in der Schichtdicke aufgebracht, die der späteren Stegdicke entspricht. Auf die Stegschicht 2" wird ferner eine fotoaktive Schicht 4' aufgebracht, die nach entsprechender fotolithografischer Behandlung eine Ätzmaske für den nasschemischen Ätzprozess bildet.

Der nass-chemische Ätzprozess bewirkt jedoch auch ein seitliches Ätzen der Stegschicht 2' bzw. ein Unterätzen der fotoaktiven Schicht 4'. Das bedeutet, dass auch unterhalb der fotoaktiven Schicht 4' die Stegschicht 2" teilweise entfernt wird. Die mit derartigen Verfahren hergestellten Stege der Gitterstruktur weisen daher ein trapezförmiges Profil auf, wobei an der Trapezbasis relativ scharfe Kanten entstehen, die negative Auswirkungen auf die Funktionsweise des Stents zur Folge haben können. Insbesondere besteht die Gefahr, dass die scharfen Kanten des Trapezprofils zu einer Verletzung von Gefäßwänden oder zu einer negativen Beeinflussung der Blutströmung in einem Blutgefäß führen. Überdies weisen die mit dem nass-chemischen Ätzprozess hergestellten Stege gemäß dem aus dem Stand der Technik bekannten Verfahren eine relativ große Stegbreite, insbesondere an der Trapezbasis, auf, so dass die Flexibilität des bekannten Stents weiterhin eingeschränkt ist.

Daher wird in der eingangs genannten WO 2008 000 467 A1 ein Verfahren vorgeschlagen, das die Herstellung einer Gitterstruktur einer erhöhten Kantengenauigkeit der Stege ermöglicht. Dabei wird auf ein Substrat zunächst eine Opferschicht aufgebracht, die mit Hilfe einer fotolithografisch bearbeiteten Ätzmaske strukturiert wird. Das Strukturieren der Opferschicht erfolgt über einen Trockenätzprozess, um die hohe Kantengenauigkeit zu erreichen. In einem weiteren Schritt wird das Substrat einem nasschemischen Ätzprozess unterzogen, wodurch die Opferschicht unterätzt wird. Nach Entfernen der Ätzmaske wird anschließend das Stentmaterial auf den Schichtverbund aus Substratschicht und Opferschicht in einem Sputterprozess aufgebracht, wobei sich das Stentmaterial teilweise auf der Opferschicht und teilweise auf dem geätzten Substratmaterial anlagert. Nach Entfernen der Substratschicht, der Opferschicht und dem in die nasschemisch geätzten Bereiche der Substratschicht eingebetteten Stentmaterial bleibt die gewünschte freitragende Gitterstruktur bestehen.

Ein Nachteil des Verfahrens gemäß WO 2008 000 467 A1 besteht darin, dass das Opferschichtmaterial, das vorzugsweise Gold, Kupfer oder Chrom umfasst, nach der Entfernung nicht mehr für den Herstellungsprozess zur Verfügung steht bzw. nur durch einen aufwändigen Recyclingprozess wiederverwendet werden kann. Das bekannte Verfahren ist daher relativ kostenaufwändig. Ferner erfordert das Entfernen der Opferschicht einen zusätzlichen Verfahrensschritt, wodurch der Zeitaufwand für die Herstellung eines Stents erhöht wird.

Ein weiteres Herstellungsverfahren für einen Stent findet sich in DE 10 2006 029 831 A1.

Der Erfindung liegt daher die Aufgabe zu Grunde, das bekannte Verfahren im Hinblick auf die Wirtschaftlichkeit zu verbessern.
Erfindungsgemäß wird diese Aufgabe durch den Gegenstand des Patentanspruchs 1 gelöst.

Die Erfindung beruht auf dem Gedanken, ein Verfahren zur Herstellung eines medizinischen Funktionselements mit einer freitragenden Gitterstruktur, die miteinander verbundene, insbesondere unter einem Winkel verbundene Stege aufweist, anzugeben, wobei das Verfahren die folgenden Verfahrensschritte umfasst:
- Eine auf die Substratschicht aufgebrachte erste Schicht wird bereitgestellt.
- Die erste Schicht wird durch einen Ätzprozess strukturiert.
- Die strukturierte erste Schicht wird durch einen auf die Substratschicht wirkenden nass-chemischen Ätzprozess unterätzt.
- Eine Stegaufbauschicht wird auf die erste Schicht aufgebracht.
- Die Substratschicht wird zur Bildung der freitragenden Gitterstruktur entfernt.

Dabei bildet die erste Schicht eine Stegansatzschicht, die eine kleinere Schichtdicke als die Stegaufbauschicht aufweist und mit der Stegaufbauschicht stoffschlüssig verbunden wird derart, dass die Stegansatzschicht mit der Stegaufbauschicht gemeinsam die Stege der freitragenden Gitterstruktur bildet.

Der Kern der Weiterentwicklung besteht also darin, die Kosten des Verfahrens durch Vermeidung von Ausschussmaterial bzw. Opfermaterial zu reduzieren. Dies wird dadurch erreicht, dass auf die Substratschicht direkt eine Stegansatzschicht aufgebracht wird, die mit der Stegaufbauschicht gemeinsam die Stege der herzustellenden Gitterstruktur bildet. Auf diese Weise trägt die Stegansatzschicht zur Stegdicke bei, wodurch das Herstellungsverfahren beschleunigt wird. Insgesamt wird also durch die Verwendung der Stegansatzschicht die Wirtschaftlichkeit des erfindungsgemäßen Verfahrens gegenüber herkömmlichen Verfahren erhöht.

Indem die erste Schicht bzw. Stegansatzschicht zur Bildung der Stege beiträgt, bietet das erfindungsgemäße Verfahren den weiteren wesentlichen Vorteil, dass die Selektivität zwischen der Stegansatzschicht und der Substratschicht erhöht wird. Das bedeutet, dass das Material der Stegansatzschicht bzw. das Ätzmittel derart wählbar ist, dass beim Unterätzen der Stegansatzschicht eine Beschädigung der Stegansatzschicht vermieden wird. Auf diese Weise wird eine besonders hohe Kantengenauigkeit erreicht.

Die Stegansatzschicht kann das gleiche Material wie die Stegaufbauschicht aufweisen, wodurch Prozesszeit und Materialkosten eingespart werden. Die Einsparung ergibt sich insbesondere durch die Verwendung weniger, insbesondere nur zweier, unterschiedlicher Materialien, beispielsweise ein erstes Material für die Substratschicht und ein gemeinsames zweites Material für die die Stege bildende Stegansatz- und Stegaufbauschicht.

Bei einer bevorzugten Ausführungsform des erfindungsgemäßen Verfahrens wird die Stegansatzschicht durch einen nass-chemischen Ätzprozess strukturiert. Das nass-chemische Ätzen hat den Vorteil, dass die Prozessgeschwindigkeit gegenüber einem Trockenätzverfahren erhöht ist. Dabei hat sich gezeigt, dass durch das Aufbringen einer weiteren Materialschicht, insbesondere der Stegaufbauschicht, auf die Stegansatzschicht, der Einfluss des seitlichen Ätzens auf die Kantengenauigkeit vernachlässigbar ist. Insbesondere bei einer relativ kleinen Schichtdicke der Stegansatzschicht wird eine hohe Kantengenauigkeit erreicht.

Ein weiterer Vorteil des nass-chemischen Ätzprozesses besteht darin, dass die Herstellungskosten und Herstellungszeit reduziert werden, da auf zusätzliche Einrichtungen, beispielsweise Anlagen für das Trockenätzen, und die darin durchgeführten Prozessschritte, verzichtet werden kann.

Vorzugsweise wird durch den auf die Substratschicht wirkenden nass-chemischen Ätzprozess ein Fangbecken bereitgestellt, in dem beim Aufbringen der Stegaufbauschicht Ausschussmaterial angeordnet wird. Dadurch wird auf einfache Weise die Strukturierung der herzustellenden Gitterstruktur erreicht. Insbesondere ermöglicht das Fangbecken eine einfache Herstellung der Zellen der Gitterstruktur.

Das Fangbecken kann eine Ätztiefe aufweisen, die kleiner als die Schichtdicke des Ausschussmaterials bzw. der Stegaufbauschicht ist. Durch die relativ geringe Ätztiefe wird das Herstellungsverfahren weiter beschleunigt. Überdies ermöglicht eine geringe Ätztiefe die Herstellung einer Gitterstruktur mit kleiner Stegbreite, da zwischen der Ätztiefe und der Ausdehnung der seitlichen Ätzung bzw. Unterätzung ein geometrischer Zusammenhang besteht.

Indem das Fangbecken eine kleinere Ätztiefe aufweist als die Stegaufbauschicht, wird die seitliche Ätzung der Substratschicht bzw. das Unterätzen der Stegansatzschicht außerdem begrenzt, so dass die Substratschicht unterhalb der Stegansatzschicht eine ausreichend breite Stützrippe bildet, die die Stegansatzschicht abstützt.

Besonders bevorzugt ist ein Verhältnis zwischen Ätztiefe und Schichtdicke der Stegaufbauschicht von weniger als 1:1, insbesondere weniger als 1:2, insbesondere weniger als 1:4, insbesondere weniger als 1:10.

Bei einer bevorzugten Ausführungsform des erfindungsgemäßen Verfahrens wird die Stegansatzschicht und/oder die Stegaufbauschicht durch einen physikalischen Abscheideprozess, insbesondere einen Sputterprozess, gebildet. Derartige Prozesse zeichnen sich durch eine hohe Prozessgenauigkeit und eine hohe Prozessgeschwindigkeit aus.

Auf die Stegaufbauschicht kann wenigstens eine weitere stegbildende Schicht aufgebracht werden. Das erfindungsgemäße Verfahren eignet sich also zur Herstellung von Gitterstrukturen, deren Stege schichtweise aufgebaut sind. Dabei ist es möglich, dass die weitere stegbildende Schicht ein von der Stegaufbauschicht verschiedenes Material aufweist, so dass Eigenschaften unterschiedlicher Werkstoffe vorteilhaft kombinierbar sind.

Vorzugsweise umfasst die weitere stegbildende Schicht bioabsorbierbares oder ein röntgensichtbares Material, insbesondere Tantal. Das Verfahren ermöglicht somit eine einfache Herstellung von medizinischen Funktionselementen, insbesondere Implantaten, die sich durch eine erhöhte Röntgensichtbarkeit auszeichnen. Dabei kann die Biokompatibilität des Implantats dadurch gewährleistet werden, dass die röntgensichtbare Schicht zwischen zwei biokompatiblen Schichten eingebettet wird.

Bei einer bevorzugten Ausführungsform des erfindungsgemäßen Verfahrens umfassen die Stegansatzschicht und die Stegaufbauschicht eine Nickel-Titan-Legierung. Eine Nickel-Titan-Legierung bzw. allgemein ein Formgedächtnismaterial eignet sich besonders zur Herstellung von medizinischen Funktionselementen, die selbstexpandierende Eigenschaften aufweisen. Überdies sind Nickel-Titan-Legierungen biokompatibel.

Die Stegansatzschicht kann vor dem Ätzen mit einer fotoaktiven Schicht versehen werden, die einem lithografischen Prozess unterzogen wird und eine Ätzmaske in Form der herzustellenden Gitterstruktur bildet. Die Strukturierung der Stegansatzschicht und die daraus resultierende Herstellung der Gitterstruktur werden auf diese Weise erleichtert und beschleunigt.

Die Erfindung wird im Folgenden anhand von Ausführungsbeispielen unter Bezugnahme auf die beigefügten schematischen Zeichnungen näher erläutert. Darin zeigen
- Fig. 1: Einen Längsschnitt durch einen Stent im implantierten Zustand;
- Fig. 2a, 2b: einen Ausschnitt einer Gitterstruktur eines Stents gemäß dem Stand der Technik;
- Fig. 3: einen Teilquerschnitt durch die Gitterstruktur eines Stents bei der Herstellung in einem Verfahren gemäß dem Stand der Technik;
- Fig. 4 bis 6: jeweils einen Teilquerschnitt durch die Gitterstruktur eines Stents in einem Verfahrensschritt des erfindungsgemäßen Verfahrens nach einem bevorzugten Ausführungsbeispiel; und
- Fig. 7 bis 10: jeweils einen Querschnitt durch einen Steg eines Stents bei der Herstellung nach dem erfindungsgemäßen Verfahren gemäß einem bevorzugten Ausführungsbeispiel.

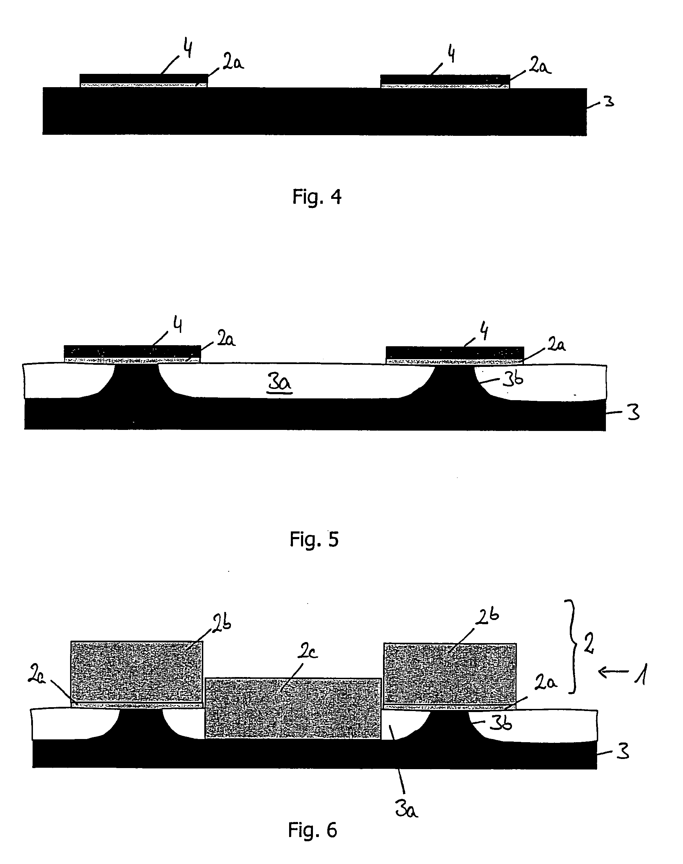

Die Fig. 4 bis 6 zeigen ausgewählte Verfahrensschritte des erfindungsgemäßen Verfahrens nach einem bevorzugten Ausführungsbeispiel. Das Verfahren basiert darauf, eine Stegansatzschicht 2a mit einer relativ kleinen Schichtdicke auf eine Substratschicht 3 aufzubringen, wobei die Stegansatzschicht 2a das die herzustellenden Stege 2 der Gitterstruktur 1 bildende Material aufweist. Die Stegansatzschicht 2a wird vorzugsweise durch ein physikalisches Abscheideverfahren, insbesondere durch Sputtern, auf die Substratschicht 3 aufgebracht. Die Strukturierung der Stegansatzschicht 2a erfolgt durch eine Ätzmaske, die durch eine fotoaktive Schicht 4 gebildet ist. Dabei wird die fotoaktive Schicht 4 bzw. eine Fotolackschicht auf die Stegansatzschicht 2a aufgebracht und durch einen fotolithografischen Prozess, beispielsweise eine entsprechende Belichtung, strukturiert.

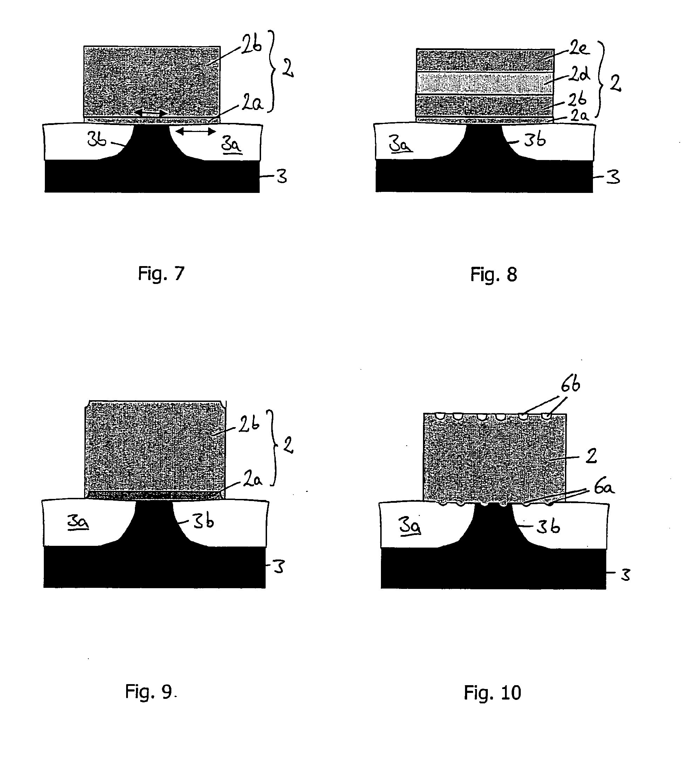

In einem weiteren, nicht dargestellten Verfahrensschritt, wird die Stegansatzschicht 2a nass-chemisch geätzt, wobei die zwischen der strukturierten fotoaktiven Schicht 4 gelegenen Bereiche der Stegansatzschicht 2a entfernt werden. Durch die relativ kleine Schichtdicke der Stegansatzschicht 2a wird erreicht, dass der Grad der Unterätzung der fotoaktiven Schicht 4 durch den nass-chemischen Ätzprozess vernachlässigbar klein ist. Dazu weist die Stegansatzschicht 2a vorzugsweise eine Schichtdicke zwischen 0,025 µm und 5 µm, insbesondere zwischen 0,05 µm und 4,5 µm, insbesondere zwischen 0,1 µm und 4 µm, insbesondere zwischen 0,25 µm und 3 µm, insbesondere zwischen 0,5 µm und 2,5 µm, insbesondere zwischen 0,8 µm und 1,5 µm, auf. Überdies werden, wie in Fig. 9 dargestellt, die seitlich geätzten Bereiche der Stegansatzschicht 2a in einem späteren Verfahrensschritt durch die Stegaufbauschicht 2b ergänzt bzw. aufgefüllt, so dass die hergestellte Gitterstruktur 1 Stege 2 mit einer hohen Kantengenauigkeit aufweist.

Nach der Strukturierung der Stegansatzschicht 2a wird in einem weiteren nasschemischen Ätzprozess die Substratschicht 3 in den zuvor anhand der Ätzmaske freigelegten Bereichen geätzt. Dabei erfolgt ein Unterätzen der Stegansatzschicht 2a, wodurch sich Stützrippen 3b der Substratschicht 3 bilden, die die Stegansatzschicht 2a und die fotoaktive Schicht 4 tragen. Zwischen den Stützrippen 3b werden durch den Ätzprozess Fangbecken 3a ausgebildet.

Um eine ausreichend stabile freitragende Gitterstruktur 1 zu erhalten, ist eine Erhöhung der Stegdicke über die Schichtdicke der Stegansatzschicht 2a hinaus erforderlich. Dazu wird in einem weiteren Verfahrensschritt gemäß Fig. 6 nach Entfernen der fotoaktiven Schicht 4 eine Stegaufbauschicht 2b auf der Stegansatzschicht 2a bzw. der Substratschicht 3 abgeschieden. Vorzugsweise erfolgt das Auftragen der Stegaufbauschicht 2b durch einen Sputterprozess. Die Stegaufbauschicht 2b umfasst dabei das gleiche Material wie die Stegansatzschicht 2a. Vorzugsweise umfassen die Stegansatzschicht 2a und die Stegaufbauschicht 2b eine Nickel-Titan-Legierung, die die Stege 2 der herzustellenden Gitterstruktur bildet. Die Schichtdicke der Stegaufbauschicht 2b ist signifikant größer als die Schichtdicke der Stegansatzschicht 2a. Insbesondere weist die Stegaufbauschicht 2b vorzugsweise eine Schichtdicke auf, die wenigstens 5 µm, insbesondere wenigstens 8 µm, insbesondere wenigstens 10 µm, insbesondere wenigstens 12 µm, insbesondere wenigstens 20 µm, insbesondere wenigstens 30 µm, insbesondere wenigstens 35 µm, insbesondere wenigstens 40 µm, insbesondere wenigstens 45 µm, insbesondere wenigstens 50 µm, beträgt.

Beim Aufbringen der Stegaufbauschicht 2b wird ein Teil des Schichtmaterials als Ausschussmaterial 2c im Fangbecken 3a der Substratschicht 3 abgelegt. Das Ausschussmaterial 3c trägt also nicht zum Aufbau der Gitterstruktur bei.

Das Fangbecken 3a weist dabei vorzugsweise eine Tiefe bzw. Ätztiefe auf, die kleiner als die Stegdicke ist, die sich aus der Summe der Schichtdicken der Stegaufbauschicht 2b und der Stegansatzschicht 2a ergibt. Das Verhältnis zwischen der Ätziefe des Fangbeckens 3a und der Schichtdicke der Stegaufbauschicht 2b kann vorzugsweise weniger als 1:1, insbesondere weniger als 1:2, insbesondere weniger als 1:4, insbesondere wenigere als 1:10, betragen. Beispielsweise beträgt die Ätztiefe 5 µm, wobei die Schichtdicke der Stegaufbauschicht 2b 50 µm beträgt. Es ist insbesondere zweckmäßig, wenn das Verhältnis zwischen Ätztiefe und Stegdicke derart angepasst ist, dass beim Unterätzen der Stegansatzschicht 2a eine ausreichend breite Stützrippe 3b zur Stützung der Stegansatzschicht 2a bereitgestellt wird.

Dadurch ist es möglich, die Unterätzung bzw. seitliche Ätzung des Fangbeckens 3a zu reduzieren. Das Verhältnis zwischen der Tiefenätzung und der seitlichen Ätzung beträgt aus physikalischen Gründen im Allgemeinen 1:1. Bei einer Stegbreite von 15 µm und einer Ätztiefe von 5 µm erfolgt daher eine seitliche Ätzung von 5 µm. Die Substratschicht 3 bildet dabei eine Stützrippe 3b mit einer Restbreite von 5 µm, die die Stegansatzschicht 2a während des weiteren Auftragens der Stegaufbauschicht 2b stützt.

Wenn die Ätztiefe kleiner als die Schichtdicke der Stegaufbauschicht 2b bzw. des Ausschussmaterials 2c ist, bildet des Ausschussmaterials 2c mit der Stegaufbauschicht 2b einen Überlappungsbereich (Fig. 6), der vorteilhafterweise eine zusätzliche, insbesondere seitliche, Abstützung für die Stegansatzschicht 2a bildet. Da sich das Ausschussmaterial 2c an den senkrechten Wänden der Stegaufbauschicht 2b schlecht anlagert, ist das Ausschussmaterial 2c in einem späteren Verfahrensschritt leicht von der Gitterstruktur bzw. dem Steg 2, insbesondere der Stegansatzschicht 2a und der Stegaufbauschicht 2b, trennbar.

Der Zusammenhang zwischen Breite der Stützrippe 3b und Ätztiefe des Fangbeckens 3a ist in Fig. 7 illustriert. Darin ist zu erkennen, dass sich der nass-chemische Ätzprozess im Wesentlichen radial ausbreitet. Das bedeutet, dass aus der Substratschicht 3 in alle Ausbreitungsrichtungen im gleichen Maße Material entfernt wird. Die Ätztiefe bzw. die Tiefe des Fangbeckens 3a entspricht somit der Tiefe der seitlichen Unterätzung der Stegansatzschicht 2a. Um die Stegbreite der herzustellenden Gitterstruktur 1 möglichst klein zu halten und gleichzeitig eine entsprechende Breite der Stützrippe 3b vorzusehen, so dass die Stegansatzschicht 2a ausreichend gestützt ist, sind die vorgenannten Verhältnisse daher besonders vorteilhaft.

Fig. 8 zeigt ein weiteres Ausführungsbeispiel des erfindungsgemäßen Verfahrens, wobei auf die Stegaufbauschicht 2b, die eine signifikant größere Schichtdicke aufweist als die Stegansatzschicht 2a, zwei weitere Stegaufbauschichten aufgebracht werden. Dabei können die Stegaufbauschichten von der Stegaufbauschicht 2b bzw. Stegansatzschicht 2a verschiedene Materialien umfassen. Die weiteren Stegaufbauschichten können beispielsweise eine röntgensichtbare Schicht 2d umfassen, die zwischen der ersten Stegaufbauschicht 2b und einer Deckschicht 2e angeordnet ist. Die röntgensichtbare Schicht 2d umfasst vorzugweise Tantal oder ein anderes röntgensichtbares Material, beispielsweise Niob, Platin, Gold oder Legierungen mit derartigen Materialien. Dabei kann die Deckschicht 2e das gleiche Material aufweisen, wie die Stegaufbauschicht 2b bzw. die Stegansatzschicht 2a. Die Schichtdicken der einzelnen Stegaufbauschichten können variieren. Insbesondere kann die röntgensichtbare Schicht 2d eine signifikant kleinere Schichtdicke aufweisen wie die Stegaufbauschicht 2b und die Deckschicht 2e.

Es ist auch möglich, dass die weiteren Stegaufbauschichten teilweise bioabsorbierbare Materialien umfassen. Beispielsweise können eine oder mehrere weitere Stegaufbauschichten Magnesium, Eisen oder Legierungen mit Magnesium und/oder Eisen umfassen. Die Stege 2 können also aus mehreren Stegaufbauschichten 2b bzw. aus einem Schichtverbund gebildet sein, der Schichten aus röntgensichtbaren und/oder bioabsorbierbaren Materialien und/oder Formgedächtnismaterialien umfasst. Die Stegansatzschicht 2a kann ebenfalls ein röntgensichtbares oder bioabsorbierbares Material umfassen. Es ist also möglich, dass beispielsweise die Stegansatzschicht 2a und/oder die Deckschicht 2e jeweils ein bioabsorbierbares Material aufweisen, so dass die Wandstärke der Stege 2 nach der Implantation im zeitlichen Verlauf abnimmt. Mit dem erfindungsgemäßen Verfahren hergestellte Stents weisen dadurch hinsichtlich der Strömungsverhältnisse im Blutgefäß verbesserte Eigenschaften auf.

Mit dem erfindungsgemäßen Verfahren ist gemäß einem weiteren Ausführungsbeispiel, das in Fig. 10 dargestellt ist, möglich, die Innen- und Außenflächen der Stege 2 zu profilieren bzw. strukturieren. Dabei bewirkt das gleichmäßige Auftragen der Stegansatzschicht 2a bzw. Stegaufbauschicht 2b durch das Sputterverfahren bzw. durch allgemein das physikalische Abscheideverfahren, dass in die Substratschicht 3 eingebrachte Strukturen auf die Oberfläche der Stegansatzschicht 2a und anschließend auf die Stegaufbauschicht 2b übertragen werden. Vorzugsweise werden Wellenprofile, Vorsprünge 6a oder Hohlräume 6b in die Substratschicht 3 eingebracht, die korrespondierend in der Oberfläche der Stegaufbauschicht 2b erscheinen. Das Profilieren bzw. Strukturieren der Substratschicht 3 kann durch einen Ätzprozess, insbesondere einen nass-chemischen Ätzprozess, oder ein anderes Materialabtragendes Verfahren, beispielsweise durch einen Laser, erfolgen.

Bei dem Ausführungsbeispiel gemäß Fig. 10 weist die Unterseite des gebildeten Stegs 2, also die Unterseite der Stegansatzschicht 2a, ein Profil mit Vorsprüngen 6a auf, das sich insbesondere durch eine verbesserte Endothelialisierung auszeichnet. Das bedeutet, dass bei Verwendung der hergestellten Gitterstruktur 1 als Stent die Strömungsverhältnisse des Blutes auf dieser Seite der Stege 2, insbesondere der Innenseite der Gitterstruktur 1, verbessert sind und somit ein Anhaften von Endothelzellen begünstigen. Die korrespondierend zu den Vorsprüngen 6a an der Unterseite des Stegs 2 gebildeten Hohlräume 6b an der Oberseite des Stegs 2 können beispielsweise als Medikamentendepot genutzt werden. Andere Profilierungen bzw. Strukturierungen der Stege 2 sind möglich. Beispielsweise können Hohlräume 6b und Vorsprünge 6a alternierend auf beiden Seiten der Stege 2 vorgesehen sein.

Die Erfindung eignet sich allgemein zur Herstellung von medizinischen Funktionselementen, insbesondere Stents, Clot Retrievern, (Blut-)Filtern und Implantaten. Vorteilhaft bei der Herstellung derartiger Mikrosysteme für die Medizintechnik mit dem erfindungsgemäßen Verfahren ist die hohe Kantengenauigkeit bei kleinen Stegbreiten. Dieser Vorteil kommt beispielsweise auch bei der Herstellung von Elektroden bzw. Mikroelektroden zum Tragen, die insbesondere implantierbar sein können.

### Bezugszeichenliste

- 1, 1': Gitterstruktur
- 2, 2': Steg
- 2": Stegschicht
- 3, 3': Substratschicht
- 4, 4': fotoaktive Schicht

- 2a: Stegansatzschicht
- 2b: Stegaufbauschicht
- 2d: röntgensichtbare Schicht
- 2e: Deckschicht
- 3a: Fangbecken
- 3b: Stützrippe
- 3c: Ausschussmaterial
- 5: Blutgefäß
- 5a: erster Gefäßabschnitt
- 5b: zweiter Gefäßabschnitt
- 6a: Vorsprung
- 6b: Hohlraum
- α: Rautenwinkel im Ruhezustand
- β: Rautenwinkel im gestreckten Zustand

## Patentansprüche

1. Verfahren zur Herstellung eines medizinischen Funktionselements mit einer freitragenden Gitterstruktur, die miteinander verbundene Stege (2) aufweist, bei dem
- eine erste Schicht auf eine Substratschicht (3) aufgebracht wird,
- die erste Schicht durch einen Ätzprozess strukturiert wird,
- die strukturierte erste Schicht durch einen auf die Substratschicht (3) wirkenden nass-chemischen Ätzprozess unterätzt wird,
- eine Stegaufbauschicht (2b) auf die erste Schicht aufgebracht wird und
- die Substratschicht (3) zur Bildung der freitragenden Gitterstruktur entfernt wird,
**dadurch gekennzeichnet, dass**
die erste Schicht eine Stegansatzschicht (2a) bildet, die eine kleinere Schichtdicke als die Stegaufbauschicht (2b) aufweist und mit der Stegaufbauschicht (2b) stoffschlüssig verbunden wird derart, dass die Stegansatzschicht (2a) mit der Stegaufbauschicht (2b) gemeinsam die Stege (2) der freitragenden Gitterstruktur bildet.

2. Verfahren nach Anspruch 1,
**dadurch gekennzeichnet, dass**
die Stegansatzschicht (2a) das gleiche Material wie die Stegaufbauschicht (2b) aufweist.

3. Verfahren nach Anspruch 1 oder 2,
**dadurch gekennzeichnet, dass**
die Stegansatzschicht (2a) durch einen nass-chemischen Ätzprozess strukturiert wird.

4. Verfahren nach wenigstens einem der Ansprüche 1 bis 3,
**dadurch gekennzeichnet, dass**
durch den auf die Substratschicht (3) wirkenden nass-chemischen Ätzprozess ein Fangbecken (3a) bereitgestellt wird, in dem beim Aufbringen der Stegaufbauschicht (2b) Ausschussmaterial (2c) angeordnet wird.

5. Verfahren nach Anspruch 4,
**dadurch gekennzeichnet, dass**
das Fangbecken (3a) eine Ätztiefe aufweist, die kleiner als die Schichtdicke des Ausschussmaterials (2c) und/oder der Stegaufbauschicht (2b) ist.

6. Verfahren nach Anspruch 5,
**dadurch gekennzeichnet, dass**
das Verhältnis zwischen Ätztiefe und der Schichtdicke der Stegaufbauschicht (2b) weniger als 1:1, insbesondere weniger als 1:2, insbesondere weniger als 1:4, insbesondere weniger als 1:10, beträgt.

7. Verfahren nach wenigstens einem der Ansprüche 1 bis 6,
**dadurch gekennzeichnet, dass**
die Stegansatzschicht (2a) und/oder die Stegaufbauschicht (2b) durch einen physikalischen Abscheideprozess, insbesondere einen Sputterprozess, gebildet werden.

8. Verfahren nach wenigstens einem der Ansprüche 1 bis 7,
**dadurch gekennzeichnet, dass**
auf die Stegaufbauschicht (2b) wenigstens eine weitere stegbildende Schicht (2d, 2e) aufgebracht wird.

9. Verfahren nach Anspruch 8,
**dadurch gekennzeichnet, dass**
die weitere stegbildende Schicht (2d, 2e) ein von der Stegaufbauschicht (2b) verschiedenes Material aufweist.

10. Verfahren nach Anspruch 8 oder 9,
**dadurch gekennzeichnet, dass**
die weitere stegbildende Schicht (2d, 2e) ein bioabsorbierbares oder röntgensichtbares Material, insbesondere Tantal, umfasst.

11. Verfahren nach wenigstens einem der Ansprüche 1 bis 10,
**dadurch gekennzeichnet, dass**
die Stegansatzschicht (2a) und die Stegaufbauschicht (2b) eine Nickel-Titan-Legierung umfassen.

12. Verfahren nach wenigstens einem der Ansprüche 1 bis 11,
**dadurch gekennzeichnet, dass**
die Stegansatzschicht (2a) vor dem Ätzen mit einer fotoaktiven Schicht (4) versehen wird, die einem lithographischen Prozess unterzogen wird und eine Ätzmaske in Form der herzustellenden Gitterstruktur bildet.

## Claims

1. A method for manufacturing a medical functional element comprising a self-supporting lattice structure having interconnected webs (2), in which
- a first layer is applied to a substrate layer (3),
- the first layer is structured by an etching process,
- the structured first layer is underetched by a wet chemical etching process that acts on the substrate layer (3),
- a web add-on layer (2b) is applied to the first layer and
- the substrate layer (3) is removed to form the self-supporting lattice structure,
**characterized in that**
the first layer forms a web base layer (2a) which has a smaller layer thickness than the web add-on layer (2b) and is joined to the web add-on layer (2b) by material bonding, such that the web base layer (2a) together with the web add-on layer (2b) jointly forms the webs (2) of the self-supporting lattice structure.

2. The method according to Claim 1,
**characterized in that**
the web base layer (2a) has the same material as the web add-on layer (2b).

3. The method according to Claim 1 or 2,
**characterized in that**
the web base layer (2a) is structured by a wet chemical etching process.

4. The method according to at least one of Claims 1 to 3,
**characterized in that**
a collecting basin (3a), in which scrap material (2c) is arranged when applying the web add-on layer (2b), is supplied by the wet chemical etching process, which acts on the substrate layer (3).

5. The method according to Claim 4,
**characterized in that**
the collecting basin (3a) has a smaller etching depth than the layer thickness of the scrap material (2c) and/or the web add-on layer (2b).

6. The method according to Claim 5,
**characterized in that**
the ratio between the etching depth and the layer thickness of the web add-on layer (2b) is less than 1:1, in particular less than 1:2, in particular less than 1:4, in particular less than 1:10.

7. The method according to at least one of Claims 1 to 6,
**characterized in that**
the web base layer (2a) and/or the web add-on layer (2b) is/are formed by a physical deposition process, in particular a sputtering process.

8. The method according to at least one of Claims 1 to 7,
**characterized in that**
at least one additional web-forming layer (2d, 2e) is applied to the web add-on layer (2b).

9. The method according to Claim 8,
**characterized in that**
the additional web-forming layer (2d, 2e) has a material different from the web add-on layer (2b).

10. The method according to Claim 8 or 9,
**characterized in that**
the additionally web-forming layer (2d, 2e) comprises a bioabsorbable or radiopaque material, in particular tantalum.

11. The method according to at least one of Claims 1 to 10,
**characterized in that**
the web base layer (2a) and web add-on layer (2b) comprise a nickel-titanium alloy.

12. The method according to at least one of Claims 1 to 11,
**characterized in that**
the web base layer (2a) is provided with a photoactive layer (4) before etching, which is subjected to a lithographic process and forms an etching mask in the form of the lattice structure to be produced.

## Revendications

1. Procédé de fabrication d'un élément fonctionnel médical doté d'une structure de grille en porte-à-faux et qui présente des traverses reliées entre elles (2), dans lequel
- une première couche est appliquée sur une couche de substrat (3),
- la première couche est structurée par un procédé de gravure chimique humide
- la première couche structurée est gravée par dessous par un procédé de gravure agissant sur la couche de substrat (3),
- une couche d'augmentation de traverses (2b) est appliquée sur la première couche et
- la couche de substrat (3) est éliminée pour former la structure de grille en porte-à-faux,
**caractérisé en ce que**
la première couche forme une couche de base de traverses (2a) qui a une épaisseur de couche inférieure à celle de la couche d'augmentation de traverses (2b) est et est reliée par assemblage de matière à la couche d'augmentation de traverses (2b) de manière à ce que la couche de base de traverses (2a) forme les traverses (2) de la structure de grille en porte-à-faux en commun avec la couche d'augmentation de traverses (2b).

2. Procédé selon la revendication 1,
**caractérisé en ce que**
la couche de base de traverses (2a) contient le même matériau que la couche d'augmentation de traverses (2b).

3. Procédé selon la revendication 1 ou 2,
**caractérisé en ce que**
la couche de base de traverses (2a) est structurée par un procédé de gravure chimique humide.

4. Procédé selon au moins une des revendications 1 à 3,
**caractérisé en ce**
**qu'**est créé par le procédé de gravure chimique humide agissant sur la couche de substrat (3) un bassin récupérateur (3a) dans lequel du matériau rebuté (2c) est disposé lors de l'application de la couche d'augmentation de traverses (2b).

5. Procédé selon la revendication 4,
**caractérisé en ce que**
le bassin récupérateur (3a) a une profondeur de gravure qui est inférieure à l'épaisseur de couche du matériau rebuté (2c) et/ou de la couche d'augmentation de traverses (2b).

6. Procédé selon la revendication 5,
**caractérisé en ce que**
le rapport entre la profondeur de gravure et l'épaisseur de couche de la couche d'augmentation de traverses (2b) est de moins de 1 à 1, en particulier moins de 1 à 2, en particulier moins de 1 à 4, en particulier moins de 1 à 10.

7. Procédé selon au moins une des revendications 1 à 6,
**caractérisé en ce que**
la couche de base de traverses (2a) et/ou la couche d'augmentation de traverses (2b) sont formées par un procédé physique de dépôt, en particulier un procédé de pulvérisation.

8. Procédé selon au moins une des revendications 1 à 7,
**caractérisé en ce**
**qu'**au moins une autre couche constituante de traverses (2d, 2e) est appliquée sur la couche d'augmentation de traverses (2b).

9. Procédé selon la revendication 8,
**caractérisé en ce que**
l'autre couche constituante de traverses (2d, 2e) présente un matériau différent de celui de la couche d'augmentation de traverses (2b).

10. Procédé selon la revendication 8 ou 9,
**caractérisé en ce que**
l'autre couche constituante de traverses (2d, 2e) comprend un matériau bioabsorbable ou visible par radiographie, en particulier du tantale.

11. Procédé selon au moins une des revendications 1 à 10,
**caractérisé en ce que**
la couche de base de traverses (2a) et la couche d'augmentation de traverses (2b) comprennent un alliage de nickel et titane.

12. Procédé selon au moins une des revendications 1 à 11,
**caractérisé en ce que**
la couche de base de traverses (2a), avant d'être gravée, est pourvue d'une couche photoactive (4) qui est soumise à un procédé lithographique et constitue un masque de gravure sous forme de la structure de grille à réaliser.
